(19) Europäisches Patentamt
European Patent Office
Office européen des brevets

(11) Veröffentlichungsnummer: **0 439 766 A2**

(12) # EUROPÄISCHE PATENTANMELDUNG

(21) Anmeldenummer: **90124284.2**

(22) Anmeldetag: **14.12.90**

(51) Int. Cl.5: **C07D 207/27**, C07D 207/277, A61K 31/40

(30) Priorität: **31.01.90 DD 337440**

(43) Veröffentlichungstag der Anmeldung: **07.08.91 Patentblatt 91/32**

(84) Benannte Vertragsstaaten: **AT CH DE ES FR GB IT LI SE**

(71) Anmelder: **ARZNEIMITTELWERK DRESDEN GmbH**
**Wilhelm-Pieck-Strasse 35**
**O-8122 Radebeul(DE)**

(72) Erfinder: **Zenker, Lothar, Dr.**
**Sachsenstrasse 27**
**O-8122 Radebeul(DE)**
Erfinder: **Wunderlich, Helmut, Dr.**
**Westendring 22**
**O-8027 Dresden(DE)**
Erfinder: **Lohmann, Dieter, Dr.**
**Hoflössnitzstrasse 30**
**O-8122 Radebeul(DE)**
Erfinder: **Rostock, Angelika, Dr.**
**Böttgerstrasse 36**
**O-8023 Dresden(DE)**
Erfinder: **Siegemund, Christine**
**Hauptstrasse 12**
**O-8256 Weinböhla(DE)**
Erfinder: **Valdman, Artur Viktorovic, Prof. Dr.**
**Leningradskij prospekt 71, Wohnung 122**
**125 057 Moskau(SU)**
Erfinder: **Voronina, Tatjana Aleksandrovna, Prof. Dr.**
**Ul. Usaceva 2, Wohnung 165**
**165 107 000 Moskau(SU)**
Erfinder: **Glozman, Oleg Michajlovic**
**3-ja Rybinskaja ul. 21, Wohnung 186**
**107 113 Moskau(SU)**
Erfinder: **Garibova, Taisija Leonovna**
**Ul Krasnojarskaja 10, Wohnung 243**
**107 589 Moskau(SU)**
Erfinder: **Mescerjakova, Larisa Michajlovna**
**Ul vasilevskogo 7, Wohnung 101**
**123 098 Moskau(SU)**
Erfinder: **Zmurenko, Ljudmila Alaksandrovna**
**Belovezskaja ul 15, Wohnung 13**
**121 353 Moskau(SU)**
Erfinder: **Seredenin, Sergej Borisovic, Prof. Dr.**
**Ul Podmoskovnaja 14, Wohnung 32**
**123 362 Moskau(SU)**
Erfinder: **Rozancev, Grigorij Grigorevic, Dr.**
**Ul Udalcova 13, Wohnung 35**
**117 415 Moskau(SU)**
Erfinder: **Rachmankulova, Ilmira Chamidovna**
**Konakovshki prospekt 19, Wohnung 15**
**125 565 Moskau(SU)**

(74) Vertreter: **Puchberger, Rolf, Dipl. Ing. et al**
**Patentanwälte, Dipl. Ing. Georg Puchberger Dipl. Ing. Rolf Puchberger Dipl. Ing. Peter Puchberger Singerstrasse 13 Postfach 55**
**A-1010 Wien(AT)**

(54) **Verfahren zur Herstellung von N-Acyl-4-phenyl-pyrrolidin-2-onen mit cerebroprotektiver Wirkung.**

(57) N-Acyl-4-phenyl-pyrrolidin-2-one, 4-Phenyl-pyrrolidin-2-on, reaktionsfähiges Derivat einer Carbonsäure, Umsetzung, N-Acylaminobuttersäurederivat, Cyclisierung, physiologisch verträgliche Substanzen, cerebroprotektive Wirkung, nootrope Wirkung, pharmazeutisches Präparat

Verfahren zur Herstellung von N-Acyl-4-phenyl-pyrrolidin-2-onen mit cerebroprotektiver Wirkung

Die Erfindung betrifft N-Acyl-4-phenyl-pyrrolidin-2-one, die auf Grund ihrer ausgeprägten cerebroprotektiven Wirkung in der Humanmedizin zur Prophylaxe und Behandlung cerebraler Funktionsstörungen angewendet werden können.

Erfindungsgemäß werden diese Verbindungen hergestellt, indem man 4-Phenyl-pyrrolidin-2-one mit einem reaktionsfähigen Derivat einer Carbonsäure umsetzt oder N-Acylaminobuttersäurederivate cyclisiert.

EP 0 439 766 A2

# VERFAHREN ZUR HERSTELLUNG VON N-ACYL-4-PHENYL-PYRROLIDIN-2-ONEN MIT CEREBROPROTEKTIVER WIRKUNG

## Anwendungsgebiet der Erfindung

Die vorliegende Erfindung betrifft Verfahren zur Herstellung von N-Acyl-4-phenyl-pyrrolidin-2-onen der allgemeinen Formel

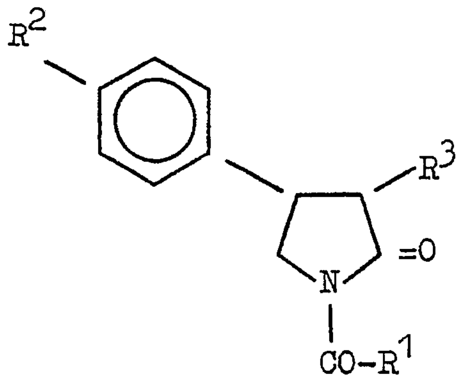

(I),

worin

- $R^1$ eine Alkylgruppe mit 1 bis 9 C-Atomen, die geradkettig oder verzweigt, gesättigt oder ungesättigt sein kann, eine Aryl-, Aralkyl- oder Heteroarylgruppe, die durch ein Halogenatom oder eine Alkoxygruppe mit 1 bis 3 C-Atomen im Alkylrest substituiert sein kann, oder die p-Chlorphenoxymethylgruppe,
- $R^2$ Wasserstoff, Halogen oder eine Alkoxygruppe mit 1 bis 3 C-Atomen im Alkylrest und
- $R^3$ Wasserstoff oder eine Carbalkoxygruppe mit 1 bis 3 C-Atomen im Alkylteil

bedeuten.

Die Verbindungen der allgemeinen Formel I sind potentielle Pharmaka mit ausgeprägter cerebroprotektiver Wirkung bei hoher Verträglichkeit, die in der Humanmedizin, vorzugsweise in der Prophylaxe und Behandlung cerebraler Funktionsstörungen, angewendet werden können.

## Charakteristik des bekannten Standes der Technik

In der DD-PS 119 229 wird die Herstellung von 4-(Polyalkoxyphenyl)-pyrrolidin-2-onen beschrieben, die zentralsedierende, neuroleptische Eigenschaften haben.

Das in der JP-PS 70 16 692 genannte, am N-Atom nichtsubstituierte 4-(4-Chlorphenyl)-pyrrolidin-2-on weist antikonvulsive Wirkungen auf.

In der US-PS 4,443,616 wird die Herstellung von Pyrrolidin-2-onen aus 3-Pyrrolin-2-onen beansprucht, wobei im weiteren Umfang nicht näher ausgeführte ZNS-aktive Wirkungen erwähnt werden.

In 4-Position unsubstituierte N-Acyllactame zeigen neben diversen ZNS-Effekten auch antiamnestische Wirkungen im Tierexperiment (JP-PS 3 246 328). Die cerebroprotektiven Eigenschaften dieser Verbindungen sind - wie eigene Versuche zeigten - im Vergleich zu den erfindungsgemäßen Verbindungen der allgemeinen Formel I nur schwach ausgeprägt.

Weiterhin sind aus der Patentliteratur (DD-PS 256 694) N-Dipropylacetyllactame mit 5-, 6- oder 7-gliedrigem Lactamring bekannt geworden, die neben antikonvulsiven Wirkungen im Tierexperiment antihypoxische und nootrope Effekte aufweisen. Die erfindungsgemäßen neuen Verbindungen der allgemeinen Formel I zeigen demgegenüber eine entscheidende und für die therapeutische Anwendung am Menschen vorteilhafte Verschiebung des pharmakologischen Spektrums bei gleichzeitig verringerter Toxizität.

Im Vergleich zu weiteren, schon seit längerer Zeit in die medizinische Praxis eingeführten nootropen Wirkstoffen lassen sich ebenfalls Vorzüge der erfindungsgemäßen Verbindungen I aufzeigen.

So ist die antiamnestische Wirkung von I in entsprechenden Tierexperimenten um mehr als das einhundertfache stärker als die der Standard-Nootropika Piracetam und Meclofenoxat.

## Ziel der Erfindung

Ziel der Erfindung ist die Herstellung neuer Verbindungen mit wertvollen pharmakologischen Eigenschaften.

Darlegung des Wesens der Erfindung

Der Erfindung liegt die Aufgabe zugrunde, neue cerebroprotektiv wirksame und gut verträgliche Stoffe für die Anwendung in der Humanmedizin herzustellen.

Erfindungsgemäß wird dies dadurch ermöglicht, daß man ein 4-Phenyl-pyrrolidin-2-on der allgemeinen Formel

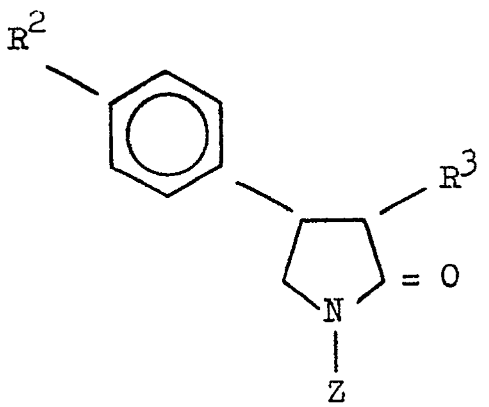

(II) ,

worin

| | |
|---|---|
| Z | Wasserstoff oder ein Alkalimetall bedeutet und |
| $R^2$ und $R^3$ | die oben angegebene Bedeutung haben, mit einem reaktionsfähigen Derivat einer Carbonsäure, die den Kohlenwasserstoffrest $R^1$ enthält, in an sich bekannter Weise umsetzt. |

Als Kohlenwasserstoffreste $R^1$ kommen geradkettige oder verzweigte, gesättigte oder ungesättigte Alkylgruppen mit 1 bis 9 C-Atomen in Betracht, sowie Aryl-, Aralkyl- oder Heteroarylgruppen, die durch ein Halogenatom oder eine Alkoxygruppe mit 1 bis 3 C-Atomen im Alkylrest substituiert sein können. Außerdem kann $R^1$ den p-Chlorphenoxymethylrest darstellen.

Geeignete Alkylgruppen sind zum Beispiel Methyl, Ethyl, Propyl, Isopropyl, Butyl, Isobutyl, tert.-Butyl, Pentyl, 2-Methylbutyl, 2,2-Dimethylpropyl, Hexyl, Heptyl, 4-Heptyl, Octyl, Nonyl, 1,2-Dimethylheptyl.

Die Alkylgruppen können auch ungesättigt sein und Vinyl, 1-Propenyl, 2-Propenyl usw. bedeuten.

Die Arylgruppe ist vorzugsweise ein Phenylrest, als Aralkylgruppe eignen sich vorzugsweise Cinnamoyl- bzw. Tolylreste. Für die Heteroarylgruppe stehen beispielsweise N-Heterocyclen wie der Nicotinylrest.

Der Rest $R^1$ kann auch einfach oder mehrfach substituiert sein, beispielsweise durch Halogen, insbesondere Fluor, Chlor, Brom, durch Hydroxygruppen, z. B. 4-Hydroxypropyl, oder Aminogruppen, bei denen der Stickstoff gegebenenfalls durch Alkylgruppen mit vorzugsweise 1 bis 5 C-Atomen mono- oder disubstituiert sein kann oder Bestandteil eines 5- bis 7-gliedrigen Ringes ist.

Als Alkoxygruppen kommen vor allem Methoxy, Ethoxy sowie n- oder Isopropoxygruppen in Frage.

Als reaktionsfähige Carbonsäurederivate eignen sich besonders die Carbonsäureanhydride, vorzugsweise der niederen Carbonsäuren, und die Carbonsäurehalogenide, wobei den technisch leicht zugänglichen Carbonsäurechloriden ein Vorzug einzuräumen ist.

Weiterhin ist die Umsetzung von II (Z = H) mit aktivierten Carbonsäureestern zu den erfindungsgemäßen Verbindungen I möglich. Beispielsweise kann der Carbonsäurepentachlorphenylester mit Erfolg eingesetzt werden.

Eine besondere Ausführungsform der Erfindung besteht darin, ein Salz von II, wobei Z für ein Alkalimetall steht, mit einem Carbonsäurehalogenid zur Umsetzung zu bringen. Zur Bildung des Alkalisalzes werden in an sich bekannter Weise Alkalimetalle, Alkaliamide oder andere reaktionsfähige Alkaliderivate wie Alkalialkoholate oder Alkalihydroxide verwendet.

Für die Beschleunigung und Vollständigkeit der Umsetzung von II mit Carbonsäurehalogeniden erweist es sich als vorteilhaft, Halogenwasserstoffacceptoren wie Triethylamin, Pyridin, N,N-Dimethylanilin oder Alkalicarbonate zu verwenden.

Die Umsetzung von II mit dem reaktionsfähigen Carbonsäurederivat kann sowohl in Gegenwart als auch in Abwesenheit inerter organischer Lösungsmittel erfolgen. Beispielsweise verwendet man aliphatische oder

aromatische Kohlenwasserstoffe wie Benzen, Toluen oder Xylen, halogenierte aliphatische oder aromatische Kohlenwasserstoffe wie Dichlormethan, Chloroform oder Chlorbenzen, Ester wie Methyl- oder Ethylacetat, Ether wie Diethylether oder Dioxan, Ketone wie Aceton oder Methylethylketon oder Formamide wie Dimethylformamid oder Gemische derselben.

Die Reaktionstemperaturen können in einem größeren Bereich variiert werden. Im allgemeinen arbeitet man unter Erwärmen bis zur Siedetemperatur des jeweiligen Lösungsmittels oder Lösungsmittelgemisches. Wenn man als Halogenwasserstoffacceptor Pyridin einsetzt, kann man durch den bekannten, aktivierenden Effekt von Pyridin auf die Reaktivität von Acylhalogeniden schon bei wesentlich erniedrigten Temperaturen wie Temperaturen in einem Bereich von -40 °C bis Raumtemperatur befriedigende Umsetzungen erzielen.

Desweiteren kann zur Herstellung von I ($R^3$ = H) die Cyclisierung von N-Acylaminobuttersäurederivaten der allgemeinen Formel

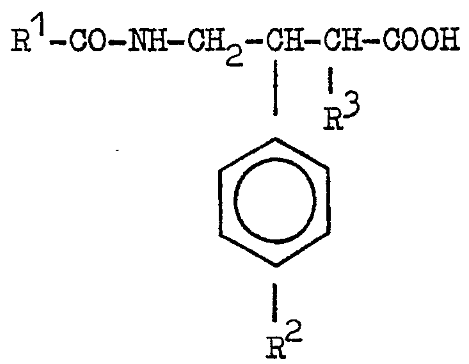

(III),

worin $R^1$ und $R^2$ die oben angegebene Bedeutung haben und $R^3$ für Wasserstoff steht, durchgeführt werden.

Die Ringschlußreaktion kann sowohl thermisch, vorzugsweise bei Temperaturen oberhalb des Schmelzpunktes der Verbindungen III, durch Anwendung von wasserbindenden Mitteln, wie Carbonsäureanhydriden, insbesondere Acetanhydrid, oder durch Bildung von instabilen Carbonsäurehalogeniden von III mittels Halogenierungsmitteln, beispielsweise durch Umsetzung von III mit Thionylchlorid, die unter Halogenwasserstoffaustritt zu I weiterreagieren, erreicht werden.

Verfahrensgemäß erhältliche Verbindungen I können in üblicher Weise in andere Verbindungen der Formel I überführt werden. So kann man beispielsweise den Rest $R^2$ durch gezielte Reaktionen austauschen. Halogenverbindungen lassen sich beispielsweise durch Behandeln von I ($R^2$ = H) mit Chlor oder Brom, vorteilhaft in Gegenwart einer Lewissäure, z. B. von Eisen(III)-chlorid, herstellen.

Ausgangsstoffe II können beispielsweise hergestellt werden, indem man einen entsprechenden 4-Amino-3-(4'-$R^2$-phenyl)-buttersäurealkylester durch Behandeln mit Schwefelsäure oder ethanolischer Salzsäure, zu den entsprechenden 4-(4'-$R^2$-phenyl)-pyrrolidin-2-onen cyclisiert. 4-Amino-3-(4'-$R^2$-phenyl)-buttersäure läßt sich in an sich bekannter Weise leicht thermisch, gegebenenfalls in Gegenwart eines hochsiedenden Lösungsmittels unter Dehydratisierung cyclisieren. II ($R^3$ = $COOC_2H_5$) wird üblicherweise durch Reduktion von 2-Carbethoxy-3-(4'-$R^2$-phenyl)-4-nitrobuttersäureethylestern, vorzugsweise durch Hydrierung an Räney-Nickel, erhalten.

Die Verbindungen der allgemeinen Formel I stellen ölige oder kristalline, allgemein farblose bis schwach gelbliche Stoffe dar, die sich in Wasser nicht lösen.

Die Verbindungen I besitzen psychotrope Wirkungen, die sie für eine Verwendung als Cerebroprotektiva geeignet machen. Sie schützen das Hirn vor schädigenden Einflüssen und verbessern seine Leistungsfähigkeit. So ist die antiamnestische Wirkung einiger Verbindungen in einem Lerntest 1000fach stärker als die von Piracetam und 300fach stärker als die von Meclofenoxat oder Ca-Valproat (Tabelle 1). Solche Untersuchungen werden für gedächtnisverbessernde Substanzen z. B. von SARA, S. und DAVID-REMACLE, M.: Psychopharmacologia 1974, 36, 59 oder HOFFMANN, W. und ROSTOCK, A.: Pharmazie 5 1983, 38, 12, 869 beschrieben. Weiterhin normalisieren die Verbindungen die durch verschiedene schädigende Faktoren (chronischer Alkoholverbrauch, Hirnischämie) verschlechterte Lernleistung von Ratten. Ein weiterer Vorteil der Verbindungen ist, daß sie keine sedierende Wirkung haben. So tritt z. B. am Drehstab bis zur Dosis 2 000 mg/kg keine Beeinträchtigung des Koordinationsvermögens auf.

In ähnlicher Weise erweist es sich als günstig, daß die erfindungsgemäßen Verbindungen I im Vergleich zu bekannten nootrop wirksamen Verbindungen wie Carbamazepin, Dipropylessigsäure oder N-Dipropylacetyl-pyrrolidin-2-on keine nennenswerten antikonvulsiven Eigenschaften besitzen.

Die experimentellen Befunde über fehlende sedierende Wirkungen sowie fehlende oder nur schwach ausgeprägte antikonvulsive Eigenschaften von I sind insofern von besonderer Wichtigkeit, weil sich die therapeutische Anwendung der Stoffe bei Hirnleistungsstörungen im allgemeinen über einen längeren Zeitraum erstreckt und Nebenwirkungen weitestgehend vermieden werden müssen.

Die beschriebenen Verbindungen I erweisen sich als hochverträglich. Die $LD_{50}$ im Tierexperiment liegt über 2 000 mg/kg Maus bei intraperitonealer Applikation.

In der nachstehenden Tabelle 1 sind Effektivitäts- und Toxizitätswerte von erfindungsgemäßen Verbindungen der allgemeinen Formel I zusammengestellt und in Vergleich zu Werten gesetzt, die für bekannte nootrop wirksame Verbindungen der Literatur gelten.

Tabelle 1

| Substanz (siehe Beispiele) | antiamnestisch wirksame Dosis (mg/kg Ratte ip.) | $LD_{50}$ (mg/kg Maus ip.) |
|---|---|---|
| 1 | 0,1 | 1 632 |
| 2 | 100 | > 2 000 |
| 3 | 100 | > 2 000 |
| 4 | 1 | > 2 000 |
| 5 | 1 | > 2 000 |
| 6 | 0,1 | > 2 000 |
| Piracetam | 100 | > 2 000 |
| Aniracetam | 100 | 1 000 |
| Meclofenoxat | 30 | 856 |
| Ca-Valproat | 30 | 455 |

Die neuen Verbindungen der allgemeinen Formel I können als pharmazeutische Präparate Verwendung finden, welche eine therapeutisch wirksame Menge der Wirksubstanz, gegebenenfalls zusammen mit anderen anorganischen oder organischen, festen oder flüssigen, pharmazeutisch verwendbaren Trägerstoffen, enthalten.

Man kann I in Form von Tabletten, Dragees, Kapseln, Suppositorien, Lösungen, Suspensionen oder Emulsionen verabreichbaren Präparaten verwenden.

Die pharmazeutischen Zubereitungen, die, wenn erwünscht, weitere pharmakologisch wirksame Stoffe enthalten können, werden in üblicher Weise nach bekannten Methoden, z. B. mittels konventioneller Misch-, Granulier-, Dragier-, Lösungs- oder Lyophilisierungsverfahren hergestellt und enthalten von etwa 0,1 % bis 100 % des Wirkstoffes.

Ausführungsbeispiele

Beispiel 1

Eine Lösung aus 32,4 g (0,2 Mol) 4-Phenylpyrrolidin-2-on in 300 ml Toluen und 35,8 g (0,22 Mol) Dipropylacetylchlorid läßt man 20 h am Rückfluß sieden, wobei HCl-Gas entweicht. Nach Abkühlen auf Raumtemperatur wäscht man mit einer gesättigten $K_2CO_3$-Lösung und zweimal mit Wasser, trocknet über $CaCl_2$, treibt das Lösungsmittel im Vakuum ab und destilliert den Rückstand an der Ölpumpe.

N-Dipropylacetyl-4-phenyl-pyrrolidin-2-on (Substanz 1; $Kp_2$: 163 - 165 °C) wird als schwach gelbliche, ölige Flüssigkeit erhalten.

Ausbeute: 50 g = 87 % der Theorie.

$C_{18}H_{25}NO_2$

IR (Film): 1740 (C=O), 1690 (C=O)

In analoger Weise wurde N-Dipropylacetyl-4-(4-chlorphenyl)-pyrrolidin-2-on (Substanz 2; $Kp_{0,5}$: 200 - 202 °C) als gelbliches Öl in 79%iger Ausbeute hergestellt.

$C_{18}H_{24}NO_2Cl$

IR (Film): 1740 (C=O), 1690 (C=O)

Beispiel 2

Zu einem Gemisch aus 16,1 g (0,1 Mol) 4-Phenylpyrrolidin-2-on, 19 g (0,11 Mol) 4-Methoxybenzoylchlorid und 150 ml Dioxan tropft man 11,1 g (0,11 Mol) Triethylamin und erwärmt 2 h zum Rückfluß. Nach Abkühlen auf Raumtemperatur gießt man die Reaktionsmischung in Wasser, wobei N-(4-Methoxybenzoyl)-4-phenyl-pyrrolidin-2-on (Substanz 3) auskristallisiert. Man saugt das Rohprodukt ab und wäscht mit Wasser. Durch Auskochen in der 10fachen Menge Alkohol und anschließende Umkristallisation aus Toluen erhält man die Substanz 3 als farbloses Kristallisat vom Schmp. 147,5 - 149 °C.
Ausbeute: 19 g = 64,3 % der Theorie.

$C_{18}H_{17}NO_2$

IR (KBr): 1750 (C=O), 1660 (C=O)

Beispiel 3

19,6 g (0,1 Mol) 4-(4-Chlorphenyl)-pyrrolidin-2-on werden in 150 ml Pyridin gelöst und die Lösung auf 0 °C abgekühlt. Dann tropft man unter Rühren 21 g (0,12 Mol) 4-Methoxybenzoylchlorid zu, entfernt das Kühlbad und rührt 3 h bei Raumtemperatur. Die Reaktionsmischung wird in 750 ml 2N HCl geschüttet, wobei N-(4-Methoxybenzoyl)-4-(4-chlorphenyl)-pyrrolidin-2-on (Substanz 4) auskristallisiert. Man saugt den Niederschlag ab, wäscht mit Wasser, trocknet und kristallisiert aus der 6fachen Menge Toluen um.
Schmp. 163,5 - 165 °C.
Ausbeute: 19,5 g = 59 % der Theorie.

$C_{18}H_{16}NO_3Cl$

IR (KBr): 1750 (C=O), 1655 (C=O)

Beispiel 4

Das Gemisch aus 6,8 g (0,032 Mol)4-Chlorphenoxyacetylchlorid, 4,8 g (0,03 Mol) 4-Phenylpyrrolidin-2-on, 3 ml (0,032 Mol) Triethylamin und 30 ml Toluen rührt man 3 h bei 100 - 120 °C. Man läßt auf Raumtemperatur abkühlen und filtriert den abgeschiedenen Niederschlag ab, wäscht ihn erst mit wenig Toluen, dann mit Wasser und trocknet im Dampftrockenschrank. Man erhält N-(4-Chlorphenoxyacetyl)-4-phenyl-pyrrolidin-2-on (Substanz 7) in 76,5%iger Ausbeute. Die Verbindung schmilzt nach Umkristallisation aus Ethanol (15fache Menge) bei 129 - 130 °C.

$C_{18}H_{16}NO_3Cl$

IR (KBr): 1730 (C=O), 1710 (C=O)

Analog wurde hergestellt: N-(4-Chlorphenoxyacetyl)-4-(4-chlorphenyl)-pyrrolidin-2-on (Substanz 5).
Schmp. 181,5 - 183 °C (Chlorbenzen).
Ausbeute: 74 % der Theorie.

$C_{18}H_{15}NO_3Cl_2$

IR (KBr): 1730 (C=O), 1710 (C=O)

Analog wurde hergestellt: N-Dipropylacetyl-3-carbethoxy-4-phenyl-pyrrolidin-2-on (Substanz 10). Schmp. 50 - 51 °C (n-Hexan).
Ausbeute: 76 % der Theorie.

$C_{21}H_{29}NO_4$

IR (KBr): 1746 (C=O), 1725 (C=O), 1697 (C=O)

Beispiel 5

Ein Gemisch aus 2,9 g (0,18 Mol) 4-Phenylpyrrolidin-2-on und Nicotinsäurechlorid, hergestellt aus 2,52 g (0,02 Mol) Nicotinsäure und $SOCl_2$, 3,6 ml (0,04 Mol) Triethylamin in 15 ml Toluen erwärmt man 9 h bei 100 bis 110 °C, filtriert den sich abscheidenden Niederschlag, wäscht ihn mit Benzen und Wasser aus, trocknet ihn und gewinnt 3,1 g = 58,5 % der Theorie N-Nicotinyl-4-phenyl-pyrrolidin-2-on (Substanz 8) vom Schmp. 146 - 147 °C (Benzen).

$C_{16}H_{14}N_2O_2$

IR (KBr): 1746 (C=O), 1617 (C=O)

Analog dem Beispiel 5 wurde N-(4-Chlorbenzoyl)-4-phenyl-pyrrolidin-2-on(Substanz 9) mit einem Schmp. von 157 - 158 °C (Benzen) gewonnen.
Ausbeute: 65,4 % der Theorie.

$C_{17}H_{14}NO_2Cl$

IR (KBr): 1746 (C=O), 1671 (C=O)

Beispiel 6

58,3 g (0,25 Mol) 3-Carbethoxy-4-phenylpyrrolidin-2-on und 51 g (0,3 Mol) 4-Methoxybenzoylchlorid werden in 250 ml Toluen vorgelegt. Bei Zusatz von 33 g (0,3 Mol) Triethylamin beginnt die Bildung eines Niederschlages.
Man heizt die Suspension langsam zum Rückfluß auf und hält diese Temperatur 2 h. Danach wird abgekühlt und das Kristallisat abgesaugt. Das getrocknete Produkt wird mehrfach mit Wasser ausgerührt und anschließend in Ethanol ausgekocht. Nach Umkristallisation aus der 4fachen Menge Toluen gewinnt man farbloses N-(4-Methoxybenzoyl)-3-carbethoxy-4-phenyl-pyrrolidin-2-on (Substanz 6) vom Schmp. 137 - 139 °C (Toluen).
Durch Aufarbeiten der Mutterlaugen läßt sich eine Ausbeute von 73,5 % der Theorie realisieren.

$C_{21}H_{21}NO_5$

IR (KBr): 1750 (C=O), 1725 (C=O), 1675 (C=O)

**Patentansprüche**

**1.** Verfahren zur Herstellung von N-Acyl-4-phenyl-pyrrolidin-2-onen der allgemeinen Formel

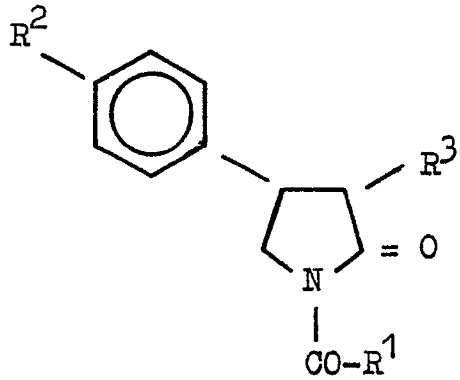

(I),

worin

$R^1$ eine Alkylgruppe mit 1 bis 9 C-Atomen, die geradkettig oder verzweigt, gesättigt oder ungesättigt sein kann, eine Aryl-, Aralkyl- oder Heteroarylgruppe, die durch ein Halogenatom oder eine Alkoxygruppe mit 1 bis 3 C-Atomen im Alkylrest substituiert sein kann, oder die p-Chlorphenoxymethylgruppe,

$R^2$ Wasserstoff, Halogen oder eine Alkoxygruppe mit 1 bis 3 C-Atomen im Alkylrest und

$R^3$ Wasserstoff oder eine Carbalkoxygruppe mit 1 bis 3 C-Atomen im Alkylteil

bedeuten, dadurch gekennzeichnet, daß man ein 4-Phenyl-pyrrolidin-2-on der allgemeinen Formel

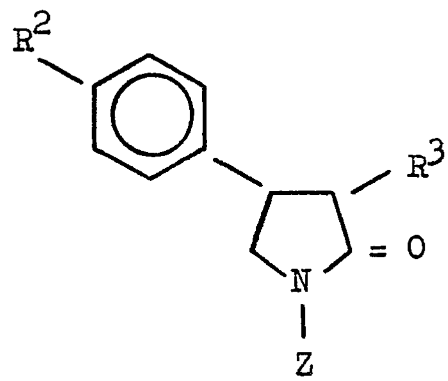

(II),

worin

Z Wasserstoff oder ein Alkalimetall bedeutet und

$R^2$ und $R^3$ die oben angegebene Bedeutung haben,

mit einem reaktionsfähigen Derivat einer Carbonsäure, die den Kohlenwasserstoffrest $R^1$ in oben angegebener Bedeutung enthält, umsetzt oder daß man ein N-Acylaminobuttersäurederivat der allgemeinen Formel

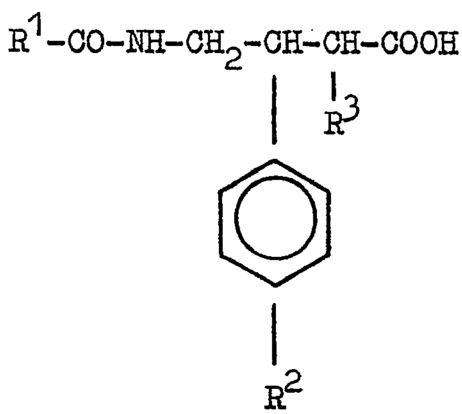

(III),

worin

$R^1$ und $R^2$ die oben angegebene Bedeutung haben und

$R^3$ für Wasserstoff steht, cyclisiert.

2. Verfahren nach Anspruch 1, dadurch gekennzeichnet, daß man die Umsetzung einer Verbindung der allgemeinen Formel II mit einem Carbonsäureanhydrid, vorzugsweise dem einer niederen Carbonsäure, durchführt.

3. Verfahren nach Anspruch 1, dadurch gekennzeichnet, daß man die Umsetzung einer Verbindung der allgemeinen Formel II mit einem Carbonsäurehalogenid, vorzugsweise mit einem Carbonsäurechlorid, durchführt.

4. Verfahren nach Anspruch 1, dadurch gekennzeichnet, daß man die Umsetzung einer Verbindung der allgemeinen Formel II, worin Z für ein Wasserstoffatom steht, mit einem aktivierten Carbonsäureester, vorteilhaft mit Carbonsäurepentachlorphenylester, durchführt.

5. Verfahren nach den Ansprüchen 1 und 3, dadurch gekennzeichnet, daß man ein Salz der Verbindung der allgemeinen Formel II, wobei Z für ein Alkalimetall steht, mit einem Carbonsäurehalogenid umsetzt.

6. Verfahren nach den Ansprüchen 1, 3 und 5, dadurch gekennzeichnet, daß man die Verbindung der allgemeinen Formel II mit einem Carbonsäurehalogenid in Gegenwart eines Halogenwasserstoffacceptors wie Triethylamin, Pyridin, N,N-Dimethylanilin oder Alkalicarbonat umsetzt.

7. Verfahren naoh den Ansprüchen 1 bis 6, dadurch gekennzeichnet, daß man die Reaktion in inerten organischen Lösungsmitteln wie aliphatischen oder aromatischen Kohlenwasserstoffen, beispielsweise Benzen, Toluen oder Xylen, halogenierten aliphatischen oder aromatischen Kohlenwasserstoffen, beispielsweise Dichlormethan, Chloroforn oder Chlorbenzen, Estern, beispielsweise Methyl- oder Ethylacetat, Ethern, beispielsweise Diethylether oder Dioxan, Ketonen, beispielsweise Aceton oder Methylethylketon, Formamiden, beispielsweise Dimethylformamid, oder einem Lösungsmittelgemisch durchführt.

8. Verfahren nach den Ansprüchen 1 bis 7, dadurch gekennzeichnet, daß man die Reaktion bei Temperaturen von -40 °C bis zur Siedetemperatur des Lösungsmittels oder Lösungsmittelgemisches durchführt.

9. Verfahren nach den Ansprüchen 1, 7 und 8, dadurch gekennzeichnet, daß man die Verbindungen der allgemeinen Formel III oberhalb des Schmelzpunktes der Verbindungen thermisch cyclisiert.

10. Verfahren nach den Ansprüchen 1, 7 und 8, dadurch gekennzeichnet, daß man die Verbindungen der allgemeinen Formel III mittels wasserbindenden Mitteln wie Carbonsäureanhydriden, beispielsweise Acetanhydrid, cyclisiert.

11. Verfahren nach den Ansprüchen 1, 7 und 8, dadurch gekennzeichnet, daß man die Verbindungen der allgemeinen Formel III durch Umsetzung mit üblichen Halogenierungsmitteln, beispielsweise Thionylchlorid, cyclisiert.

12. Verfahren nach den Ansprüchen 1 bis 11, dadurch gekennzeichnet, daß man in Verbindungen der allgemeinen Formel I die Reste $R^1$ bis $R^3$ durch Substitutionsreaktionen austauscht.

13. N-Acyl-4-phenyl-pyrrolidin-2-one der allgemeinen Formel

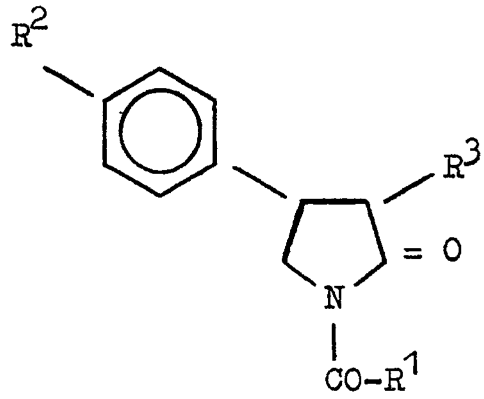

(I),

worin

- $R^1$ eine Alkylgruppe mit 1 bis 9 C-Atomen, die geradkettig oder verzweigt, gesättigt oder ungesättigt sein kann, eine Aryl-, Aralkyl- oder Heteroarylgruppe, die durch ein Halogenatom oder eine Alkoxygruppe mit 1 bis 3 C-Atomen im Alkylrest substituiert sein kann, oder die p-Chlorphenoxymethylgruppe,
- $R^2$ Wasserstoff, Halogen oder eine Alkoxygruppe mit 1 bis 3 C-Atomen im Alkylrest und
- $R^3$ Wasserstoff oder eine Carbalkoxygruppe mit 1 bis 3 C-Atomen im Alkylteil

bedeuten.

**14.** Mittel mit cerebroprotektiver Wirkung, gekennzeichnet durch einen Gehalt an einer oder mehreren Verbindungen der allgemeinen Formel I und gegebenenfalls geeigneten pharmazeutischen Trägerstoffen.